# EUROPÄISCHE PATENTSCHRIFT

(11) **EP 0 849 277 B1**
(45) Veröffentlichungstag und Bekanntmachung des Hinweises auf die Patenterteilung: **15.12.2004**
(21) Anmeldenummer: 97121847.4
(22) Anmeldetag: 11.12.1997
(51) Int. Cl.: C07K 14/62, C07K 1/18

(54) **Verfahren zur Gewinnung von Insulin durch Hochdruckflüssigchromatographie**
Process for preparation of insulin by high pressure liquid chromatography
Procédé de préparation d'insuline par chromatographie liquide à haute pression

(30) Priorität: 18.12.1996 DE 19652713
(43) Veröffentlichungstag der Anmeldung: 24.06.1998
(73) Patentinhaber: Aventis Pharma Deutschland GmbH, 65929 Frankfurt am Main (DE)
(72) Erfinder: Obermeier, Rainer, Dr., 65795 Hattersheim (DE); Ludwig, Jürgen, 63636 Brachttal (DE); Sabel, Walter, Dl., 65520 Bad Camberg (DE)

(56) Entgegenhaltungen:
- US-A- 5 101 013
- US-A- 5 245 008
- LEE W-C: "Protein separation using non-porous sorbents" JOURNAL OF CHROMATOGRAPHY B: BIOMEDICAL SCIENCES & APPLICATIONS, ELSEVIER SCIENCE PUBLISHERS, NL, Bd. 699, Nr. 1-2, 10. Oktober 1997 (1997-10-10), Seiten 29-45, XP004094987 ISSN: 1570-0232

## Beschreibung

Bei der Herstellung von rekombinanten Insulinen erfolgt die mikrobielle Biosynthese des Insulins in Bakterien wie transformierten Escherichia coli über ein einkettiges Vorläufermolekül, das neben der nativen Insulinsequenz der A- und B-Kette ein Connecting-Peptid und eine Fusionsproteinsequenz enthält. Letzteres wird aus gentechnischen Gründen am N-terminalen Ende des Gesamtproteins angeknüpft und ist dafür verantwortlich, daß das exprimierte insulinhaltige Produkt in Form von Einschlußkörperchen im transformierten E. coli anfällt. Die Aufgabe der Aufarbeitung ist es, aus diesem Fusionprotein in mehrstufigen proteinchemischen Prozeßschritten Insulin zur Verfügung zu stellen. Dabei wird in jedem Falle, wie auch bei der natürlichen Biosynthese des Insulins in der Betazelle des Pankreas, die Stufe der Faltung zum (Prä)-Proinsulin durchlaufen. Das (Prä)-Proinsulin wird durch enzymatische Spaltung (z.B. mit Trypsin) in ein Spaltungsgemisch überführt, das einen weiteren Insulinvorläufer, das Di-Arg-(B31-32)-Insulin, Mono-Arg-B31-Insulin und das Connecting-Peptid (bis zu 35 Aminosäureresten) enthält. Daneben entstehen noch Nebenprodukte, die durch die Proteaseaktivität des Trypsins erzeugt werden, wie unvollständige Intermediate, Des-Thr-B30-Insulin oder auch Arg-A0-Insulin und Präinsulin. Es ist bekannt die genannten Gemische aus Insulin und Insulinderivaten durch Normaldruck- oder Mitteldruckchromatographie an stark sauren lonenaustauschern wie S-Sepharose®, Fraktogel®TSK oder SP Trisacryl® zu trennen (US 5 101 013). Die bekannten Chromatographiematerialien sind nicht druckstabil und werden in den Chromatographiesäulen bei einem Druck oberhalb von 1 MPa komprimiert, wobei eine Trennung der insulinhaltigen Gemische dann nicht mehr möglich ist.

Ein weiteres bekanntes Verfahren zur Gewinnung von Insulin ist die Hochdruckflüssigchromatographie an lipophil modifiziertem Kieselgel (US 5 245 008, EP 0 547 544).

Kationenaustauscherreinigungsverfahren mit Normaldruck erreichen auch mit optimierten traditionellen Gelmaterialien nicht die Trennleistungen, die zur Erzielung des gewünschten Reinigungsgrades für das rekombinante Insulin benötigt werden. Die Chromatographie mit modernen präparativen Gelmaterialien, z.B. Poros 50 µm/Perseptiv, Source 30 µm/Pharmacia oder Makropep 50 µm/BioRad wird im Mitteldruckverfahren durchgeführt. Mitteldruckchromatographie mit Gelen von geringerer Teilchengröße (z.B. Source 15 µm) erbringt nur geringfügige Selektivitätsverbesserungen bezüglich der Trennleistung, so daß auch hier als letzte Hochreinigungsstufe die Hochdruckflüssigchromatographie (HPLC) zur Eliminierung geringster Verunreinigungen in den Insulinen unumgänglich bleibt. Die Nachteile einer Reverse Phase-HPLC, wie Denaturierungsgefahr des Proteins, Ausbluten der RP-Kieselgel-Phase und wenig befriedigende Cleaning in Place-Maßnahmen müssen dafür in Kauf genommen werden und erfordern einen überdurchschnittlichen Kosten- und Zeitaufwand.

In den Bestreben bessere Trenn- und Isolierverfahren zur Gewinnung von Insulin aus enzymatischen Spaltungsreaktionen bereitzustellen, wurde nun gefunden, daß sich dieses Ziel durch Chromatographie der Insulin- und Insulinderivatgemische an druckstabilen sauren Kationenaustauschern bei einem Druck von 1,1 MPa bis 40 MPa erreichen läßt. Das so gewonnene Insulin eignet sich zum direkten Einsatz ohne weitere Reinigungschritte in Injektionslösungen zur Behandlung von Diabetes mellitus.

Die Erfindung betrifft daher ein Verfahren zur Gewinnung von Insulin durch Chromatographie, das dadurch gekennzeichnet ist, daß man die Trennung mit druckstabilen sauren Kationenaustauschermaterialien bei einem Druck von 1,1 MPa bis 40 MPa durchführt.

Unter den Begriff Insulin werden Verbindungen verstanden die tierischen oder menschlichen Ursprungs sind, z.B. Humaninsulin oder Schweineinsulin, Insulinvorläufer wie Proinsuline oder Präinsuline, oder rekombinante Insuline oder Insulinderivate, die von genetisch modifizierten Mikroorganismen exprimiert werden. Insuline können auch durch chemische oder enzymatische Derivatisierung modifiziert sein, z.B. Des-Phe-B1-Insulin, Diarginininsulin, (B31, B32), Monoarginininsulin, Diphenylalanininsulin (B31,B32) (US 4 601 852), oder Gly^{A21}-Arg^{B31}-Arg^{B32}-Humaninsulin (EP 368 187).

Im erfindungsgemäßen Verfahren werden bevorzugt Insuline der Formel I eingesetzt, wobei 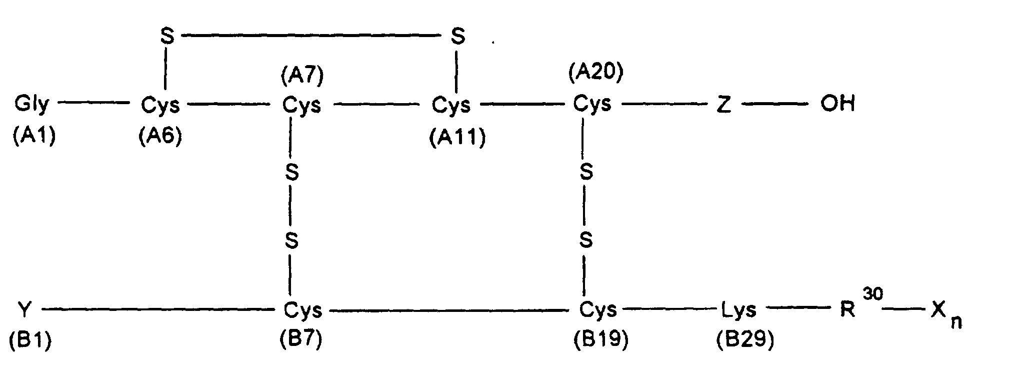
- R³⁰: für den Rest einer genetisch kodierbaren L-Aminosäure steht,
- X: für eine Hydroxygruppe, einen genetisch kodierbaren L-Aminosäurerest steht,
- n: für eine ganze Zahl von 0 bis 10 steht,
- Y: für Wasserstoffatom oder L-Phenylalaninrest steht, und
- Z: für einen genetisch kodierbaren L-Aminosäurerest steht und wobei
die Reste A2-A20 der Aminosäuresequenz der A-Kette von Humaninsulin, tierischen Insulin oder einem Insulinderivat und die Reste B2-B29 der Aminosäuresequenz der B-Kette von Humaninsulin, tierischen Insulin oder einem Insulinderivat entsprechen.

Bevorzugt werden Insuline der Formel I, wobei
- R³⁰: für einen Rest aus der Gruppe L-Alanin und L-Threonin steht,
- X: für einen L-Aminosäurerest aus der Gruppe L-Arginin, L-Lysin und L-Phenylalanin steht,
- n: für eine ganze Zahl von 0 bis 6 steht,
- Z: für einen Rest aus der Gruppe Glycin, L-Alanin, L-Serin, L-Threonin, L-Asparaginsäure und L-Glutaminsäure steht und
A1 bis A20 oder B2 bis B29 für die Aminosäuresequenz von Human-, Schweineoder Rinderinsulin steht, eingesetzt.

Insbesondere bevorzugt wird Humaninsulin und Gly^{A21}-Arg^{B31}-Arg^{B32}-Humaninsulin gewonnen.

Die Aminosäuresequenz A1 bis A20 von Humaninsulin ist:

Gly Ile Val Glu Gln Cys Cys Thr Ser Ile Cys Ser
Leu Tyr Gln Leu Glu Asn Tyr Cys (SEQ ID NO: 1)

Die Aminosäuresequenz B1 bis B29 von Humaninsulin ist:

Phe Val Asn Gln His Leu Cys Gly Ser His Leu
Val Glu Ala Leu Tyr Leu Val Cys Gly Glu Arg
Gly Phe Phe Tyr Thr Pro Lys (SEQ ID NO: 2)

Insulin kann sowohl in verhältnismäßig verunreinigtem Zustand als auch in vorgereinigter Form (z. B. durch Gelchromatographie) eingesetzt werden. Insulin ist nach mehrfacher Kristallisation und auch nach Gelchromatographie noch mit insulinähnlichen Begleitstoffen sehr ähnlichen Molekulargewichts verunreinigt, die sich bei passend gewähltem pH in ihrem Ladungszustand untereinander und vom Insulin unterscheiden, aber mit Insulin Komplexe bilden (US 4 129 560). Beispiele solcher Substanzen sind:

Desamidoinsulin, Arginin- und Diarginininsulin und Insulinethylester.

Unter dem Begriff druckstabile saure Kationenaustauschermaterialien werden beispielsweise Materialien wie ein Copolymer aus Polystyrol und Divinylbenzol, verstanden, die mit Sulfogruppen, insbesondere mit R-O-CH₂-CHOH-CH₂-O-CH₂-CHOH-CH₂-SO₃⁻-Gruppen modifiziert sind. Besonders bevorzugt sind folgende Produkte
- Source®30S, Firma Pharmacia Biotech AB, Uppsala, Schweden, druckstabile, sphärisch und poröse Materialien mit einem Partikeldurchmesser von etwa 30 µm (Downstream, No 19, (1995), Pharmacia Biotech AB, S-751 82 Uppsala, Schweden, Seiten 3-8)
- Source®15S, Firma Pharmacia Biotech AB, Uppsala, Schweden, druckstabile, sphärische und poröse Materialien mit einem Partikeldurchmesser von durchschnittlich 15 µm
- Poros 50 µm, Firma Perseptiv
- Makroprep 50 µm, Firma Biorad

Die Elutionsmittel enthalten eine Puffersubstanz, die den pH-Wert des Elutionsmittels konstant hält, Wasser und organische Lösungsmittel. Geeignete Puffersubstanzen sind in der Literatur bekannt, beispielsweise Phosphate, Alkalioder Erdalkalimetallsalze wie Kaliumacetat, Ammoniumcitrat, Natriumcitrat, -acetat, -sulfat oder -chlorid. Ferner enthalten die Elutionsmittel mit Wasser mischbare organische Lösungsmittel wie Alkohole, Ketone, Methylacetat, Dioxan oder Acetonitril. Bevorzugt werden (C₁-C₄)-Alkohole wie n- oder iso-Propanol, Methanol, Ethanol oder Butanol eingesetzt.

Für die Chromatographie beträgt die Konzentration der mit Wasser mischbaren organischen Lösungsmittel 10 bis 50 Volumenprozent, bevorzugt 20 bis 40 Volumenprozent, besonders bevorzugt 25 bis 35 Volumenprozent. Die Konzentration der Puffersubstanz beträgt etwa 1 mMol/l bis 140 mMol/l, bezogen auf Wasser als Lösungsmittel, bevorzugt 2 mMol/l bis 120 mMol/l. Weitere Zusatzstoffe die der Pufferlösung zugesetzt werden können, sind z.B. Salz, vorzugsweise physiologisch verträgliches Mineralsalz, eine oder mehrere organische Säuren wie Ameisensäure, Essigsäure, Milchsäure oder Citronensäure, vorzugsweise Milchsäure, eine Base, vorzugsweise NaOH, und/oder Konservierungsstoffe. Der bevorzugte pH-Wert der Pufferlösung beträgt etwa 2,5 bis 5,5, besonders bevorzugt von etwa 3,5 bis 4,0. Die Konzentration der organischen Säure kann in einem weiten Bereich schwanken. Vorteilhafte Mengen sind von 10 bis 100 mMol/l, bezogen auf Wasser als Lösungsmittel, bevorzugt von 25 bis 50 mMol/l.

Die Temperatur während der Chromatographie beträgt 0°C bis 50°C, vorzugsweise 15 bis 30°C, besonders bevorzugt 15 bis 20°C. Der Betriebsdruck während der Chromatographie ist weitgehend konstant. Die Chromatographie kann mit unterschiedlichem Druck durchgeführt werden, z.B. kann die Chromatographie bei einem Druck von 1,1 bis 40 MPa durchgeführt werden, insbesondere bei 1,5 bis 10 MPa. Die Eluentflüsse liegen bei 200 bis 1000 cm/h, maximal 2000 cm/h.

Die Beladung der Säulen, Chromatographie und Elution der Insuline und Insulinderivate erfolgt nach bekannten, üblichen, technischen Methoden. Die Beladung der Säule mit der zu reinigenden Insulinlösung erfolgt bevorzugt mit wäßrig-alkoholischer oder rein wäßriger Pufferlösung. Die Insulinlösung hat einen Proteinanteil von etwa 1 bis 10 %, bevorzugt 3 %.

Die Beladung des druckstabilen sauren Kationenaustauschers kann beispielsweise erfolgen, indem das Insulingemisch in einer Pufferlösung - vorzugsweise mit der zuvor beschriebenen Zusammensetzung und dem zuvor beschriebenen pH-Wert - gelöst wird und die so erhaltene Lösung mit dem druckstabilen sauren Kationenaustauscher in Kontakt gebracht wird.

Die Elutionslösung, die im Prinzip eine ähnliche Zusammensetzung wie die zuvor beschriebene Pufferlösung haben kann, weist vorzugsweise einen pH-Wert von 3,5 bis 4,0 auf. Besonders geeignet ist ein Elutionsverfahren, bei dem die Elutionslösung einen zeitlichen Salz-Konzentrationsgradienten mit vorzugsweise linearem Verlauf aufweist. Dieser Konzentrationsgradient kann z.B. angelegt werden, indem zu Beginn der Elution eine geringe Salzkonzentration (im Grenzfall gegen Null) in der Elutionslösung vorliegt und indem die Salzkonzentration während des Elutionsvorgangs gesteigert wird. Auf diese Weise kann eine besonders wirksame Trennung des Proteingemisches erreicht werden. Ein bevorzugter Salz-Konzentrationsgradient variiert von nahe 0 Mol Salz/l (zu Anfang der Elution) bis etwa 0,8 Mol Salz/l (zu Ende der Elution), besonders bevorzugt von ca. 0,10 (zu Anfang der Elution) bis ca. 0,25 Mol/l (zu Ende der Elution). Für den Salzzusatz kommen viele organische und anorganische Salze in Frage Bevorzugt sind physiologisch verträgliche Salze wie Ammonium- und Alkalisalze, besonders bevorzugt Natriumsalze, insbesondere Natriumchlorid.

Das erfindungsgemäße Trennverfahren erfolgt im Säulenverfahren. Die Temperatur, die bei der Ionenaustauscherchromatographie vorzugsweise konstant zu halten ist, kann in einem weiten Bereich variiert werden. Vorzuziehen ist ein Temperaturintervall von ca. -10°C bis ca. 50°C, insbesondere von ca. 15 bis ca. 25°C.

Die Einengung des Insulins nach der Chromatographie aus den Eluaten geschieht durch Ausfällung mit Zink-Salz oder durch Kristallisation. Dabei kann die Lösung wahlweise vorher mittels Destillation unter verminderten Druck weitgehend vom Lösungsmittel befreit oder dessen Konzentration durch Verdünnen mit Wasser reduziert werden. Auf jeden Fall sollte die Lösungsmittelkonzentration vor der Fällung oder Kristallisation bei 10 % oder darunter liegen, um den Proteingehalt im Überstand auf < 50 mg/l zu halten. Die entstandenen Insulinniederschläge lassen sich durch Dekantieren, Zentrifugieren oder Filtration isolieren und trocknen. Das erfindungsgemäße Verfahren eignet sich nicht nur zur analytischen Chromatographie, sondern auch zur präparativen Chromatographie, insbesondere wenn das erfindungsgemäße Verfahren mit einer präparativen Hochdruckflüssigchromatographie (HPLC)-Anlage durchgeführt wird.

Unter dem Begriff "präparative Chromatographie" wird ein Reinigungsverfahren verstanden mit dem Ziel pro Chromatographielauf, Reinprodukte zu gewinnen und nicht nur zu analysieren. Die Menge der Reinprodukte kann in weiten Grenzen schwanken, beispielsweise von 1 mg bis 5,0 kg, bevorzugt von 50 mg bis 2,5 kg.

In den folgenden Beispielen wird das erfindungsgemäße Verfahren im einzelnen beschrieben. Prozentangaben beziehen sich auf das Volumen, sofern nicht anders angegeben.

### Beispiel 1

- Puffer A:: 30 % n-Propanol, 50 mM Milchsäure, 0,01 M NaCl in Wasser, pH 3,5
- Sorbens:: Source® S15 µm
- Säulenabmessung:: 5 cm x 25 cm.

Eine präparative HPLC-Säule (5cmx25cm, etwa 500 ml Säulenvolumen) wird mit einer Suspension von Source S 15 µm in 50 %igem wäßrigen Ethanol gefüllt und mit Puffer A äquilibriert. Der Puffer wird dazu mit einer Flußrate von 98 ml/Minute auf die Säule gepumpt. Dabei baut sich ein Druck von 1,9 MPa auf. Die Säule ist dabei Bestandteil einer Chromatographieanlage mit Fraktionssammler und UV-Detektor (254/280 nm).
5 g des zu reinigenden rohen rekombinanten Humaninsulins werden in 500 ml des Puffers A gelöst und mit gleicher Flußrate wie oben auf die Säule gepumpt. Während einer Nachspülphase wird mit 1000 ml Puffer A erneut äquilibriert.

Zur Elution wird über ein Gradientenmischsystem niederdruckseitig ein linearer NaCl-Gradient aus Puffer A und Puffer B (Puffer B = Puffer A + 0,15 M NaCl) auf die Säule gepumpt und ein UV-Elutionsdiagramm erzielt. Das Eluat wird fraktioniert und die einzelnen Fraktionen mit Hilfe einer üblichen analytischen HPLC-Methode überprüft. Fraktionen, die der gewünschten Reinheit entsprechen werden vereinigt. Nach Verdünnung mit Wasser (1 Vol. Eluat + 2 Vol. Wasser) wird das hochgereinigte Insulin durch Kristallisation nach bekannten Verfahren als Zn²⁺-Insulin isoliert.
Ausbeute: 3,8 g Reinheit > 98 %

### HPLC-Analyse

12,5 mg Protein enthaltend Insulin werden in 25 ml des Laufmittels C (siehe Laufmittel) gelöst. Es werden 0,02 ml auf eine Hochdruckflüssigchromatographiesäule aufgetragen.
Säule: ET 250/8/4 ®Nucleosil 300-5 C18 (Macherey & Nagel, Aachen, Deutschland)
Laufmittel:

| | | |
|---|---|---|
| Stammlösung: | 41,4 g | Natriumdihydrogenphosphat * H₂O |
| | 1800 ml | bidestilliertes Wasser mit Phosphorsäure 85 % auf pH 2,5 einstellen und mit bidestillierten Wasser auf 2000 ml auffüllen. |

Laufmittel C:

| | |
|---|---|
| 500 ml | Stammlösung |
| 500 ml | Acetonitril |
| 1000 ml | bidestilliertes Wasser |

Laufmittel D:

| | |
|---|---|
| 500 ml | Stammlösung |
| 1300 ml | Acetonitril |
| 200 ml | bidestilliertes Wasser |
| 6,4 g | Natriumchlorid |

Gradient:

| Zeit | % C | % D |
|---|---|---|
| 0 min | 96 | 4 |
| 6 min | 91 | 9 |
| 15 min | 91 | 9 |
| 25 min | 85 | 15 |
| 30 min | 65 | 35 |
| 32 min | 10 | 90 |
| 35 min | 96 | 4 |
| 45 min | 96 | 4 |

Die Gradientensteigung ist so einzustellen, daß der Hauptpeak des Insulins nach 17 bis 21 min. eluiert wird.
- Temperatur:: 40°C
- Gesamtlaufzeit:: 45 min,

- Fluß:: 1 ml/min
- Detektion:: 210 nm

### Beispiel 2

Gemäß Beispiel 1 werden 5 g eines Gemisches von Des-Thr^{B30}-Humaninsulin (Insulin aus Seq ID No. 1 und No. 3 mit korrekten Cystinbrücken), Arg-B31-Humaninsulin (Insulin aus Seq ID No. 1 und No. 5 mit korrekten Cystinbrücken), und Gly^{A21}-Arg^{B31}-Arg^{B32}-Humaninsulin (Insulin aus Seq ID No. 6 und No. 4 mit korrekten Cystinbrücken) gereinigt. Das Gemisch entsteht typischerweise bei der Gewinnung von rekombinantem Insulin. Das genannte Gemisch enthält darüber hinaus auf Grund unspezifischer enzymatischer Spaltungen noch sehr geringe Mengen weiterer Verunreinigungen, die bis zum fertigen Arzneistoff abgereichert werden müssen.
Die Elution der verschiedenen Komponenten erfolgt wie in Beispiel 1. Das UV-Diagramm zeigt eine selektive Trennung der Einzelkomponenten nach steigendem isoelektrischen Punkt bei steigendem NaCl-Gradienten (Des-Thr^{B30}-Humaninsulin:I; Arg^{B31}-Humaninsulin: II; Gly^{A21}-Arg^{B31}-Arg^{B32}-Humaninsulin: III). Das fraktionierte Säuleneluat wird wie in Beispiel 1 analysiert und das gewünschte hochreine Produkt Gly^{A21}-Arg^{B31}-Arg^{B32}-Humaninsulin durch Kristallisieren isoliert. Ausbeute: 1,78 g; Reinheit > 98,5 %

### Beispiel 3

### Vergleichsbeispiel

5 g eines Gemisches wie in Beispiel 2, werden unter Bedingungen der Mitteldruckchromatographie bei 1 MPa (10 bar) und einem Eluentenfluß von 39 ml/min gereinigt. Das UV-Elutionsdiagramm zeigt eine ähnliche Trennung der Komponenten, wie in Beispiel 2. Jedoch ist bereits am Verlauf dieses Diagramms eine geringere Trennschärfe der Einzelverbindungen zu erkennen. Die HPLC-Analytik bestätigt eine deutliche Überlappung der einzelnen Insulinverunreinigungen. Das gereinigte Produkt wird durch Kristallisation isoliert. Trotz geringerer Ausbeute entspricht die Reinheit der gewünschten Spezifikation des Arzneistoffes nicht. Zu dessen Herstellung ist eine anschließende präparative HPLC-Reinigung an Reverse Phase-Kieselgel notwendig.
Ausbeute: 1,5 g Gly^{A21}-Arg^{B31}-Arg^{B32}-Humaninsulin; Reinheit: 97,6 %.

## Patentansprüche

1. Verfahren zur Gewinnung von Insulin durch Chromatographie, **dadurch gekennzeichnet, daß** die Trennung mit druckstabilen sauren Kationenaustauschermaterial bei einem Druck von 1,1 MPa bis 40 MPa erfolgt.

2. Verfahren nach Anspruch 1, **dadurch gekennzeichnet, daß** ein Insulin der Formel I, 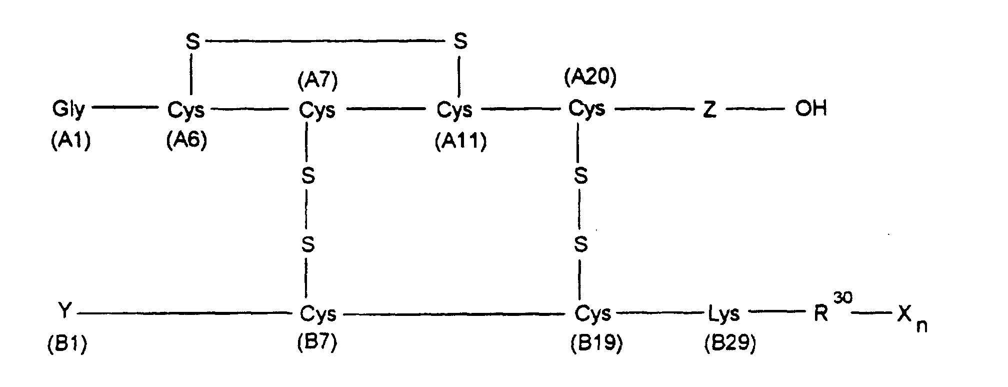 wobei
R³⁰ für den Rest einer genetisch kodierbaren L-Aminosäure, steht,
X für eine Hydroxygruppe, oder den Rest einer genetisch kodierbaren L-Aminosäure steht,
n für eine ganze Zahl von 0 bis 10, steht,
Y für Wasserstoffatom oder L-Phenylalaninrest steht, und
Z für den Rest einer genetisch kodierbaren L-Aminosäure steht, und
die Reste A2-A20 entsprechen der Aminosäuresequenz der A-Kette von Humaninsulin, tierischen Insulin oder einem Insulinderivat und die Reste B2-B29 entsprechen der Aminosäuresequenz der B-Kette von Humaninsulin, tierischen Insulin oder einem Insulinderivat, gewonnen wird.

3. Verfahren nach Anspruch 2, **dadurch gekennzeichnet, daß** ein Insulinderivat der Formel I, wobei R³⁰ für L-Alanin oder L-Threonin steht und X für eine L-Aminosäure aus der Gruppe L-Arginin, L-Lysin oder L-Phenylalanin, Z für Glycin, L-Alanin, L-Serin, L-Threonin, L-Asparaginsäure oder L-Glutaminsäure steht, n für eine ganze Zahl von Null bis 6 und A1 bis A20 oder B2 bis B29 für die Aminosäuresequenz von Human-, Schweine- oder Rinderinsulin steht, gewonnen wird.

4. Verfahren nach einem oder mehreren der Ansprüche 1 bis 3, **dadurch gekennzeichnet, daß** Humaninsulin gewonnen wird.

5. Verfahren nach einem oder mehreren der Ansprüche 1 bis 4, **dadurch gekennzeichnet, daß** die Elution mittels eines H₂O/(C₁-C₄)-Alkanol-Gemisches, das von 10 bis 50 Volumenprozent, vorzugsweise von 20 bis 40 Volumenprozent, insbesondere von 25 bis 35 Volumenprozent (C₁-C₄)-Alkanol enthält, durchgeführt wird.

6. Verfahren nach einem oder mehreren der Ansprüche 1 bis 5, **dadurch gekennzeichnet, daß** man als druckstabilen sauren Kationenaustauscher ein vernetztes Copolymer aus Polystyrol und Divinylbenzol mit Sulfogruppen verwendet.

7. Verfahren nach einem oder mehreren der Ansprüche 1 bis 6, **dadurch gekennzeichnet, daß** man eine Beladung von 5 bis 15 g Protein pro Liter Säulenvolumen des druckstabilen sauren Kationenaustauschers bei einem pH-Wert von 2,5 bis 5,5, vorzugsweise von 3,5 bis 4,0, durchführt.

8. Verfahren nach einem oder mehreren der Ansprüche 1 bis 7, **dadurch gekennzeichnet, daß** das zur Elution benutzte H₂O/(C₁-C₄)-Alkanol-Gemisch als (C₁-C₄)-Alkanol Ethanol oder Isopropanol, insbesondere Propanol, enthält.

9. Verfahren nach einem oder mehreren der Ansprüche 1 bis 8, **dadurch gekennzeichnet, daß** man den pH-Wert der Elutionslösung auf 3,5 bis 4,0 einstellt.

10. Verfahren nach einem oder mehreren der Ansprüche 1 bis 9, **dadurch gekennzeichnet, daß** die Beladungs- und Elutionslösung eine Puffersubstanz, bevorzugt auf Basis einer organischen Säure, insbesondere Milchsäure, enthält.

11. Verfahren nach einem oder mehreren der Ansprüche 1 bis 10, **dadurch gekennzeichnet, daß** mit einem Ammonium- oder Alkalisalzgradienten von 0 bis 0,8 Mol/l, insbesondere mit von 0,10 bis 0,25 Mol/l, eluiert wird.

12. Verfahren nach einem oder mehreren der Ansprüche 1 bis 11, **dadurch gekennzeichnet, daß** die Chromatographie bei einem Druck von 1,5 bis 10 MPa durchgeführt wird.

## Claims

1. A process process for isolating insulin by chromatography, wherein the separation is carried out with pressure-stable acidic cation exchange materials under a pressure of from 1.1 MPa to 40 MPa.

2. The process as claimed in claim 1, wherein an insulin of the formula I 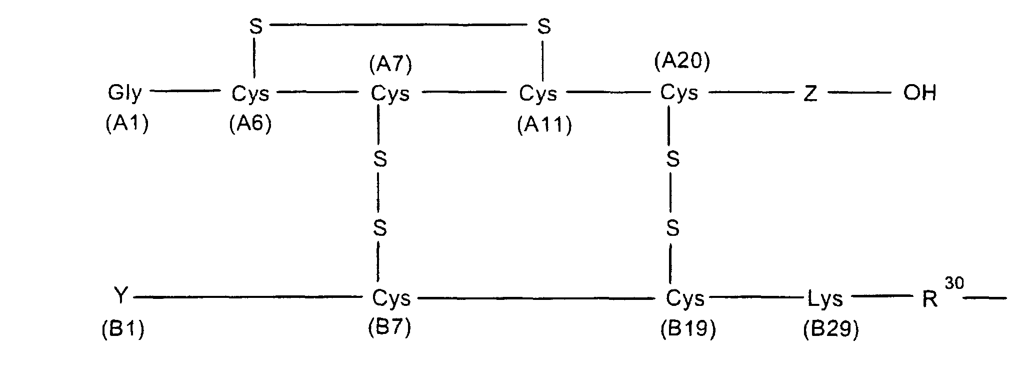 where
R³⁰ is the residue of a genetically encodable L-amino acid,
X is a hydroxyl group, or a genetically encodable L-amino acid residue,
n is an integer from 0 to 10,
Y is hydrogen atom or L-phenylalanine residue, and
Z is a genetically encodable L-amino acid residue, and
residues A2-A20 correspond to the amino acid sequence of the A chain of human insulin, animal insulin or an insulin derivative and residues B2-B29 correspond to the amino acid sequence of the B chain of human insulin, animal insulin or an insulin derivative, is isolated.

3. The process as claimed in claim 2, wherein an insulin derivative of the formula I where R³⁰ is L-alanine or L-threonine, and X is an L-amino acid from the group of L-arginine, L-lysine or L-phenylalanine, Z is glycine, L-alanine, L-serine, L-threonine, L-aspartic acid or L-glutamic acid, n is an integer from zero to 6 and A1 to A20 or B2 to B29 represents the amino acid sequence of human, porcine, or bovine insulin, is isolated.

4. The process as claimed in one or more of claims 1 to 3, wherein human insulin is isolated.

5. The process as claimed in one or more of claims 1 to 4, wherein the elution is carried out using an H₂O/(C₁-C₄) alkanol mixture which comprises from 10 to 50 percent by volume, preferably from 20 to 40 percent by volume, in particular from 25 to 35 percent by volume of (C₁-C₄) alkanol.

6. The process as claimed in one or more of claims 1 to 5, wherein a crosslinked polymer of polystyrene and divinylbenzene with sulfo groups is used as pressure-stable acidic cation exchanger.

7. The process as claimed in one or more of claims 1 to 6, wherein the pressure-stable acidic cation exchanger is loaded with from 5 to 15 g of protein per liter column volume at a pH of from 2.5 to 5.5, preferably from 3.5 to 4.0.

8. The process as claimed in one or more of claims 1 to 7, wherein the H₂O/(C₁-C₄) alkanol mixture used for the elution comprises ethanol or isopropanol, especially propanol, as (C₁-C₄) alkanol.

9. The process as claimed in one or more of claims 1 to 8, wherein the pH of the elution solution is adjusted to 3.5 to 4.0.

10. The process as claimed in one or more of claims 1 to 9, wherein the loading and elution solution comprises a buffer substance, preferably based on an organic acid, in particular lactic acid.

11. The process as claimed in one or more of claims 1 to 10, wherein elution is carried out with an ammonium or alkali metal salt gradient from 0 to 0.8 mol/l, in particular with from 0.10 to 0.25 mol/l.

12. The process as claimed in one or more of claims 1 to 11, wherein the chromatography is carried out under a pressure of from 1.5 to 10 MPa.

## Revendications

1. Procédé pour l'obtention d'insuline par chromatographie, **caractérisé en ce que** la séparation s'effectue à l'aide de substances échangeuses de cations, acides, stables à la pression, sous une pression de 1,1 MPa à 40 MPa.

2. Procédé selon la revendication 1, **caractérisé en ce qu'**on obtient une insuline de formule I, 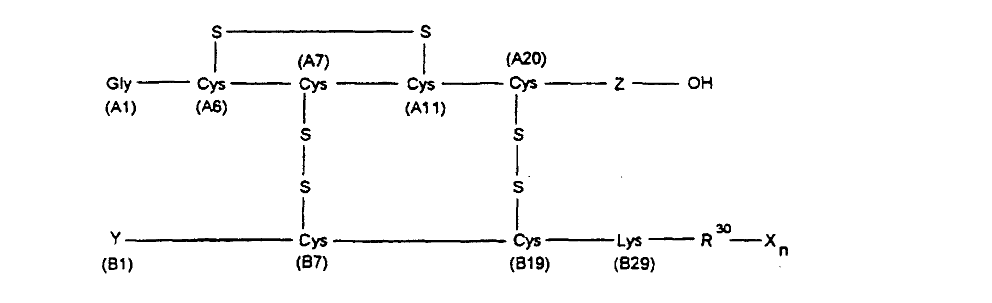 dans laquelle
R³⁰ représente le reste d'un L-aminoacide génétiquement codable,
X représente un groupe hydroxy ou le reste d'un L-aminoacide génétiquement codable,
n représente un nombre entier allant de 0 à 10,
Y représente un atome d'hydrogène ou un résidu phénylalanine, et
Z représente le reste d'un L-aminoacide génétiquement codable, et
les restes A2-A20 correspondent à la séquence d'aminoacides de la chaîne A de l'insuline humaine, d'une insuline animale ou d'un dérivé d'insuline et les restes B2-B29 correspondent à la séquence d'aminoacides de la chaîne B de l'insuline humaine, d'une insuline animale ou d'un dérivé d'insuline.

3. Procédé selon la revendication 2, **caractérisé en ce qu'**on obtient un dérivé d'insuline de formule I, dans lequel R³⁰ représente la L-alanine ou la L-thréonine et X représente un L-aminoacide choisi dans l'ensemble constitué par la L-arginine, la L-lysine et la L-phénylalanine, Z représente la glycine, la L-alanine, la L-sérine, la L-thréonine, l'acide L-aspartique ou l'acide L-glutamique, n représente un nombre entier allant de zéro à 6 et A1 à A20 ou B2 à B29 représente la séquence d'aminoacides de l'insuline humaine, porcine ou bovine.

4. Procédé selon une ou plusieurs des revendications 1 à 3, **caractérisé en ce qu'**on obtient de l'insuline humaine.

5. Procédé selon une ou plusieurs des revendications 1 à 4, **caractérisé en ce que** l'élution est effectuée au moyen d'un mélange H₂O/alcanol en C₁-C₄ qui contient de 10 à 50 % en volume, de préférence de 20 à 40 % en volume, en particulier de 25 à 35 % en volume d'alcanol en C₁-C₄.

6. Procédé selon une ou plusieurs des revendications 1 à 5, **caractérisé en ce qu'**on utilise comme échangeur de cations acide stable à la pression un copolymère réticulé de polystyrène et divinylbenzène comportant des groupes sulfo.

7. Procédé selon une ou plusieurs des revendications 1 à 6, **caractérisé en ce qu'**on fait passer une charge de 5 à 15 g de protéine par litre de volume de colonne de l'échangeur de cations acide stable à la pression, à un pH de 2,5 à 5,5, de préférence de 3,5 à 4,0.

8. Procédé selon une ou plusieurs des revendications 1 à 7, **caractérisé en ce que** le mélange H₂O/alcanol en C₁-C₄ utilisé pour l'élution contient en tant qu'alcanol en C₁-C₄ de l'éthanol ou de l'isopropanol, en particulier du propanol.

9. Procédé selon une ou plusieurs des revendications 1 à 8, **caractérisé en ce qu'**on ajuste le pH de la solution d'élution à 3,5 - 4,0.

10. Procédé selon une ou plusieurs des revendications 1 à 9, **caractérisé en ce que** la solution de charge et d'élution contient une substance tampon, de préférence à base d'un acide organique, en particulier d'acide lactique.

11. Procédé selon une ou plusieurs des revendications 1 à 10, **caractérisé en ce qu'**on effectue l'élution avec un gradient de sel d'ammonium ou de métal alcalin de 0 à 0,8 mole/l, en particulier de 0,10 à 0,25 mole/l.

12. Procédé selon une ou plusieurs des revendications 1 à 11, **caractérisé en ce que** la chromatographie est effectuée sous une pression de 1,5 à 10 MPa.
